# EUROPEAN PATENT APPLICATION

(11) **EP 4 837 653 A1**
(43) Date of publication of application: **11.11.2026**
(21) Application number: 25382461.9
(22) Date of filing: 06.05.2025
(51) Int. Cl.: B01J 21/18, B01J 23/745, B01J 35/23, B01J 35/33, B01J 35/45, B01J 35/61, B01J 37/02, B01J 37/08, C12P 5/02

(54) **CATALYTIC COMPOSITIONS FOR METHANE PRODUCTION IN ANAEROBIC DIGESTION**

(71) Applicant: Fundacion Centro Tecnologico de Componentes, 39011 Santander (ES)
(72) Inventor: AGUIRRE YAGÜE, Francisco Borja, 39011 SANTANDER (Cantabria) (ES); VADILLO ABASCAL, Jose Manuel, 39011 SANTANDER (Cantabria) (ES); ESCOBAR CLAROS, Cesar Adolfo, 39011 SANTANDER (Cantabria) (ES); RODRIGUEZ RUIZ, Alvaro, 39011 SANTANDER (Cantabria) (ES); YEDRA MARTÍNEZ, Ángel, 39011 SANTANDER (Cantabria) (ES); CASAS GARCÍA-MINGUILLÁN, Abraham, 39011 SANTANDER (Cantabria) (ES)
(74) Representative: Herrero & Asociados, S.L.

(57) **Abstract**

The present invention refers to catalytic compositions with electrostimulation capacity of methanogenic bacteria comprising a) iron-doped graphene quantum dots (Fe-GQDs) nanostructures, and b) a carbon porous structure, being the Fe-GQDs nanostructures impregnated inside the porous internal surface and the external surface of the carbon porous structure, and having the carbon porous structure a high surface area. The invention also refers to the method of obtaining said composition and its use as an additive in Anaerobic Digestion (AD) processes for methane production.

## Description

### FIELD OF THE INVENTION

The invention relates to the technical field of renewable energy and biogas production. In particular, it refers to catalytic compositions and its application in Anaerobic Digestion for the production of methane. Specifically, the present inventions is related to catalytic compositions with electrostimulation capacity of methanogenic bacteria comprising iron-doped graphene quantum dots nanostructures, and carbon porous structures.

### BACKGROUND OF THE INVENTION

Anaerobic Digestion (AD) is a widely used technology for producing biogas, predominantly methane, and serves as an effective energy recovery method from organic waste. However, several limitations in AD reduce methane production efficiency, including instability in biochemical reactions, formation of undesirable by-products, and challenges associated with the sustainable reuse of digestate by-products.

In response to these issues, the prior art has explored a range of catalytic nanomaterials, particularly nano zero-valent iron (NZVI) and carbon-based nanomaterials, to enhance methanogenesis. Despite their effectiveness, these materials present significant drawbacks, particularly regarding environmental safety, high production costs, and challenges in scaling up for industrial applications.

The following issues are associated with the use of these materials:
- Difficulties in mass production of NZVI and its cost: Given the high reactivity of NZVI particles, they react violently with air and water, leading to a highly exothermic reaction, categorizing them as pyrophoric. This poses a safety problem during their production, requiring these particles to be passivated with an iron oxide shell that stabilizes them but simultaneously reduces their effectiveness and increases production costs. According to the report by **NAVFAC,** the cost of these particles varies depending on the supplier, ranging between $50 and $150 per kilogram of material *(Arun Gavaskar Lauren Tatar Wendy Condit, NAVFAC report. CR-05-007-ENV (2005) Cost and performance report for nanoscale zero-valent iron technologies for source remediation),* making the process more expensive when scaled to industrial biogas plants.
- High concentrations of the additive in the fermentation reactor (between 5g/L and 20g/L): According to the literature, maximum increases in the final methane concentration in the biogas output of up to 18% have been reported compared to the process without adding NZVI additives *(*Yinghong F. et al. Enhanced anaerobic digestion of waste activated sludge digestion by the addition of zero-valent iron, Water Research, Volume 52, 2014, Pages 242-250). However, the required concentration of the additive in the reactor reaches 20g/L. Due to the initial cost of the additive and the environmental implications of using digester residue with iron as fertilizer for crops, where the concentration of iron increases and persists over time, the applicability of this material is very limited. However, a reduction in the additive concentration in the fermenter leads to a drastic drop in yield (2% increase with 4g/L of particles).
- Iron toxicity in crop soils: The use of leachates or byproducts from the anaerobic digester in biogas processes as fertilizers is limited by iron content. Excess iron in soil can inhibit the absorption of other nutrients, such as zinc and manganese, leading to nutritional deficiencies in plants despite high levels of iron being present *(*Li, H., et al. 2009. Effects of waterborne nano-iron on medaka (Oryzias latipes): antioxidant enzymatic activity, lipid peroxidation, and histopathology. Ecotoxicol. Environ. Saf. 72, 684-692; Khara D. Grieger, et al. Environmental benefits and risks of zero-valent iron nanoparticles (nZVI) for in situ remediation: Risk mitigation or trade-off?, Journal of Contaminant Hydrology, Volume 118, Issues 3-4, 2010, Pages 165-183*).*
- Ecotoxic effect of NZVI on aquatic organisms and evidence of neurotoxicity in rodents. *Li et al. (2009)* demonstrated the existence of oxidative stress capable of affecting embryos and adult fish species at concentrations of 500ug/L in water. Further, Phenrat et al. (Partial oxidation ("aging") and surface modification decrease the toxicity of nanosized zero-valent iron (2009) Environ. Sci. Technol. 43, 195-200*)* observed neurotoxic effects of NZVI particles on mouse neuron microglia cells. Apoptosis and cell death were also observed in response to exposure to these materials.

Nanomaterials combining biochar and iron species to promote AD processes are also known in the state of the art. For example, document WO2023197342A1 discloses the use of an iron carbonyl (Fe(CO)₅) material supported on biochar to enhance microbial interactions in AD. However, this approach does not address concerns related to iron contamination in the digestate. Similarly, CN117620199A describes a method for preparing a biochar-supported NZVI material for methane generation in AD of food waste, using sawdust as the carbon source.

Other solutions have investigated graphene-based nanomaterials to enhance the direct interspecies electron transfer (DIET) process for biomethane generation *(*Tian Tian, et al. "Nano-graphene induced positive effects on methanogenesis in anaerobic digestion, Bioresource Technology, Volume 224, 2017, Pages 41-47, ISSN 0960-8524*;* Itzel Covarrubias-García, et al. Reduced graphene oxide decorated with magnetite nanoparticles enhance biomethane enrichment, Journal of Hazardous Materials, Volume 397, 2020, 122760, ISSN 0304-3894*).* Although effective, these graphene materials do not fully address the safety, environmental compatibility, or cost-effectiveness required for large-scale AD applications.

The document US2015280248A1 describes composites comprising Graphene Quantum Dots (GQDs) functionalized with transition metals on carbon-based substrates to be applied as electrocatalysts for oxygen reduction reactions or oxygen evolution reactions. This composite have been designed to be used in a different technical field, for example, as a component of an energy storage device, such as a lithium ion battery or a supercapacitor.

In response to the problems and needs arising from the additives currently used in AD processes, and after an extensive research, the authors of the present invention have developed new catalytic nanomaterials to enhance methanogenesis. These materials are an integral and sustainable solution based on the impregnation of iron-doped graphene quantum dots (Fe- GQDs) nanostructures within the interior of highly porous carbon structures with a high specific surface area.

The specific doping with iron and the tailored integration of the GQDs in the porous carbon structure enable its novel application as a catalytic additive in AD processes for methane generation in biogas plants, enhancing the electrostimulation capacity of methanogenic bacteria.

This is an easily produced additive for the AD process, which enhances the selectivity of the biogas production process towards methane (minimizing intermediates and byproducts) while simultaneously yielding a digestate of higher quality compared to that generated in a standard biogas plant without additives.

The inherent absorption properties of its components (carbon structures and GQDs) make them additives capable of adding value to the digestate in fertilization applications, which until now have been limited by the presence of contaminants.

None of the prior art combines the catalytic benefits of graphene-based nanostructures with highly specific porous carbon structures in a manner that ensures both environmental safety and cost-effectiveness.

### BRIEF DESCRIPTION OF THE DRAWINGS

**Figure 1****:** Material obtained from the reactor filtered with a 0.22 µm filter.
**Figure 2****:** AFM image and histogram with size distribution of Fe-QGDs.
**Figure 3****:** FTIR spectrum with functional groups. Black curve for undoped GQDs and red curve for Fe-GQDs.
**Figure 4****.** Scheme of the AD process
**Figure 5****.** Graphs of chronoamperometry measurements in a) Fe₂O₃ reference, b) Fe-GQDs; c) Biochar and d) Fe-GQDs/Biochar (10:90%)

### DETAILED DESCRIPTION OF THE INVENTION

In response to current challenges in AD processes and methane production, the present invention proposes an innovative additive that offers a comprehensive and sustainable solution. This additive is based on the impregnation of iron-doped graphene quantum dots (Fe-GQDs) nanostructures within highly porous carbon structures with a high specific surface area.

In a first aspect, the present invention refers to a catalytic composition with electrostimulation capacity of methanogenic bacteria comprising:
a. Fe-GQDs nanostructures, and
b. a carbon porous structure,
wherein the Fe-GQDs nanostructures are impregnated inside the porous internal surface and the external surface of the carbon porous structure, and wherein the carbon porous structure has a high surface area ranging from 200 m²/g to 1000 m²/g.

In a particular embodiment, the Fe-GQDs nanostructures have a particle size of less than 10 nm, preferably between 2 nm and 6 nm. In a further embodiment, the Fe-GQDs are present in an amount ranging from 10% to 20% by weight, relative to the weight of the carbon porous structure. In another preferred embodiment, the Fe-GQDs nanostructures, mainly composed by carbon oxygen and nitrogen, have an iron content of less than 10% by weight, preferably between 0.1-10%, relative to the total weight of the catalytic composition.

Impregnation of the nanostructures is mainly carried out by physisorption enhanced by the presence of nitrogen moieties, so that the Fe-GQDs adhere to the internal surface of the pores and the external surface of the carbon porous structure through weak interaction forces, such as van der Waals forces, which are characterized by low adsorption energies, reversibility, and the absence of chemical bond formation.

The porous carbon structures employed in the present invention may preferably be biochar or charcoal. Biochar is a carbon-rich material obtained through the pyrolysis of biomass in a low-oxygen environment, which endows it with high porosity and a large specific surface area; these characteristics can be further enhanced through activation processes. In contrast, charcoal is produced by the pyrolysis of biomass, although the processing conditions may vary, resulting in differences in its structure and properties.

Depending on the specific application requirements, alternatives such as activated biochar, activated charcoal, or even other porous carbonaceous materials derived from agricultural or forest residues, wood, or energy crops may also be considered. These options enable the optimization of the characteristics of the carbon structures to enhance the performance of the additive in AD processes and methane production.

In a second aspect, the present invention refers to the use of the catalytic composition as an additive in AD processes for methane production.

In a third aspect, the present invention refers to a method of obtaining the catalytic composition of the invention comprising the steps of:
i. Hydrothermal synthesis of Fe-GQDs nanostructures,
ii. Generation of a carbon porous structure by pyrolysis, and
iii. Impregnation of the Fe-GQDs obtained in i) into both the internal and external surface of the carbon porous structure obtained in ii).

Both, through hydrothermal synthesis of Fe-GQDs and the generation of highly porous carbon structures by pyrolysis, highly dispersible and active materials towards the desired biogas product can be produced.

In a preferred embodiment, the hydrothermal synthesis of Fe-GQDs nanostructures involves a two-step precursor preparation. First, a reaction mixture is formed by dissolving a nitrogen source (e.g. urea), and a carbon source (e.g. trisodium citrate), in deionized water. Once homogenized, an iron salt (e.g. iron (III) chloride), is slowly added to the solution to enable doping of the GQDs with iron and nitrogen as part of functional moieties onto its structure. The resulting mixture is then subjected to hydrothermal treatment under autogenous pressure to obtain Fe-GQDs. The obtained nanostructures are subsquently filtered and dried.

Based on the proportion of iron precursor added and the subsequent purification steps, including filtration, the effective iron content in the final catalytic composition is estimated to range between 0.1% and 10% by weight, relative to the total weight of the final formulation (including the carbon porous support). In a preferred embodiment, the resulting carbon porous structure in step ii) is then ground and subjected to a surface activation step, such as ultrasonic treatment, to increase its specific surface area.

In a particular embodiment, in step iii), both components are integrated using wet techniques, based on direct mixing, or infusion methods assisted by pressure or vacuum, thus obtaining a highly active product with a large contact surface.

In a preferred embodiment, the Fe-GQDs nanostructures are incorporated into the carbon porous structure in a proportion ranging from 10% to 20% by weight, relative to the weight of the carbon porous structure (e.g. biochar). A 10% loading already enhances the Electron Donor Capacity (EDC) of the composite. Concentrations beyond 20% do not significantly improve performance, as the excess of Fe-GQDs may agglomerate on the external surface of the support, rather than being effectively dispersed within its pores.

The impregnation of Fe-GQDs into the carbon porous structure (both the external surface of the structure and the internal surface of the pores) will also guarantee the high dispersion of the additive in aqueous solution and a high specific surface, avoiding particle aggregation and losses in its catalytic activity, as occurs with other commercial additives.

In a preferred embodiment, the infusion method is a vacuum infusion up to 20 mbar in vacuum vessel that allows for greater penetration of the fluid material to be impregnated into the most inaccessible pores, achieving a greater load of nanoparticles inside the structure of the carbonaceous material.

In a fourth aspect, the present invention refers to a method to promote methane production during AD of biomass comprising: a) obtaining biomass as starting material of the AD; b) adding the catalytic composition of the invention to a biomass AD system (or reactor); and c) collecting the produced methane.

The biomass used as starting material in step a) is preferably organic waste as food, pig slurry or sludge. In particular, the process may utilize other organic residues, including animal manure from cattle, poultry, or other livestock; agricultural residues (e.g., straw, corn stover, sugar beet pulp); forest residues (such as wood, branches, and bark), dedicated energy crops (e.g., maize silage, sorghum, or miscanthus); the organic fraction of municipal solid waste; and industrial organic by-products (e.g., fruit and vegetable residues, dairy waste, or distillery by-products).

In a preferred embodiment, in step b) the catalytic composition is added to the reactor in a concentration comprised between 1 and 5 g/L.

The Fe-GQDs of the catalytic composition are lodged in suspension within the pores of the carbon porous structure, even when the material is in a dry state. During their application in the reaction medium, the Fe-GQDs are easily desorbed from the carbon structure and released by diffusion, as they are not covalently bonded. Moreover, the presence of nitrogen-based organic moieties enhances dispersibility in aqueous solution, enabling direct interaction with the bacteria and promoting their electrostimulation within the reactor. Additionally, the porous carbon structure (e.g. biochar) will remain in the medium, acting as a conductive network that promotes the connection between bacteria and Fe-GQDs

In the method of the present invention, the AD process can be implemented in either batch or semicontinuous mode, allowing it to be adapted to a wide range of operating conditions and production requirements.

In batch mode, the reactor is entirely loaded with the substrate (biomass) and the catalytic composition, enabling digestion to occur in a closed cycle with precise control over the initial conditions, which facilitates the optimization of critical parameters throughout the process. Particularly, the reactor is maintained at a temperature of 35-40°C and a pH 7-7.5, during the time required for its stabilization, preferably at least 4 days.

In contrast, in semicontinuous mode, the reactor is periodically fed with biomass while the generated products are simultaneously extracted, maintaining a dynamic equilibrium that promotes microbiological stability and a rapid response to variations in substrate loading. This operational flexibility, combined with the use of the catalytic composition, allows the process to be tailored to different scales of operation and substrate characteristics.

Advantageously, through a circular economy perspective, the present invention contemplates reusing the starting material from the AD process itself (biomass and organic material), obtaining a catalytic additive that increases the yield in the methanation stage.

Therefore, in a particular embodiment, at least one from the carbon porous structure and the Fe-GDQs nanostructures of the catalytic composition is obtained from the feedstock biomass used as starting material in the AD process.

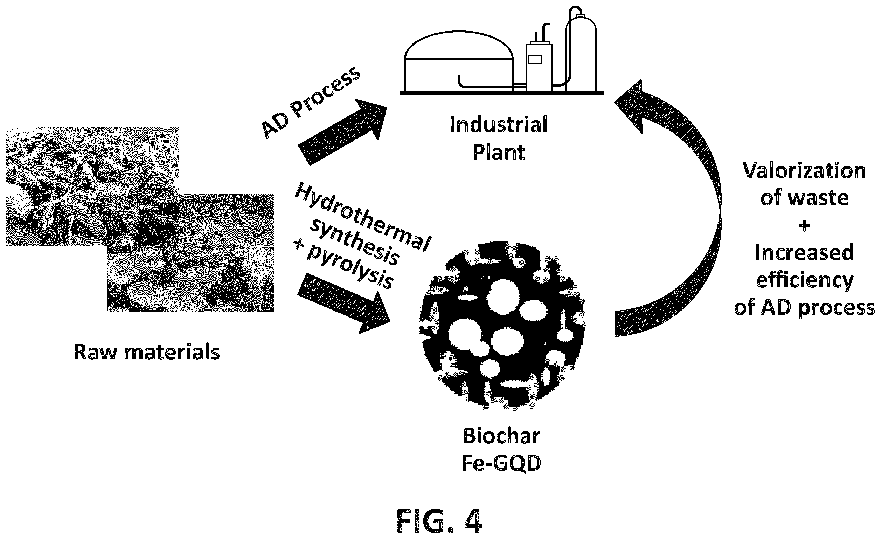

Therefore, the proposed solution is based on a strategy (Figure 4) that combines the valorization of waste from the digestor's raw material with the generation of new products that have the potential to be used both as an additive (catalytic composition) in the AD process and as a product for soil fertilization.

The present invention thus provides a combined solution that leverages the high efficiency of porous carbon structures, particularly biochar, in biogas production, while increasing efficiency of methane generation by employing Fe-GQDs nanoparticles acting as catalysts for the **DIET** process.

The advantages of the proposed innovation compared to developments included in the state of the art are the following:
- Increased efficiency due to the synergistic effect between the carbon porous structure and Fe-GQDs.
- Reduction of additive consumption in the digester (1-5 g/L) compared to the concentrations reported for the use of additives such as NZVI (10-20 g/L). Its enhanced efficiency means optimal yield is achieved with lower dosages, ultimately reducing production costs.
- Reduction of Fe content: The catalytic formulation minimizes the content of iron in the additive, enabling higher concentrations of the catalytic composition without compromising bacterial health.
- Decrease in the price of production and raw materials due to the high circularity within the AD process (cellulosic material obtained from the AD process itself).
- Production of additives based on carbon and non-critical metals, of great abundance and low environmental impact.
- Increased value of digestate because it helps minimize Fe toxicity in crop soils when by-products from AD in biogas processes are used as fertilizers, and
- Easy storage, low hazard and chemical stability in the air, unlike NZVI particles.

The porous carbon structure, combined with the uniform dispersion of Fe-GQDs, enhances catalytic efficiency at lower additive concentrations (1-5 g/L), reducing both material costs and environmental impacts. By reusing biomass from AD to create carbon porous structures or Fe-GQDs, the invention aligns with circular economy principles, reducing reliance on critical, high-impact raw materials. Finally, the low environmental impact, due to the use of raw materials from natural origin, a lower Fe content and chemical stability in air, further support the material safe use, eliminating pyrophoricity concerns and allowing digestate to be used safely as agricultural fertilizer.

### EXAMPLES

### Example 1. Synthesis of Fe-GQD/Biochar catalytic composition

### Materials

To obtain GQDs nanostructures, urea (99.5% purity, Sigma Aldrich) was used as a nitrogen source and sodium citrate dihydrate (99% purity, Panreac) as a carbon source for the synthesis of the nanostructures in the reactor. Iron doping of the nanostructures was carried out with ferric trichloride or iron (III) chloride (97% purity, Sigma Aldrich). All reactions were carried out in deionized water.

To obtain biochar, the selected starting material was woody material from fast-growing forest biomass species *(Eucalyptus Globulus)* and waste from the cleaning of heather bush areas *(Calluna Vulgaris).* The gas used to render the atmosphere of the pyrolysis reactor inert was high-purity compressed nitrogen (99.998%) ( Nippon Gases)

### Methods

### Hydrothermal synthesis stage

In 300 mL of deionized water, 30.03 g of Urea (0.5 moles) were dissolved with 43.01 g of trisodium citrate dihydrate. This kept the molar ratio of nitrogen source to carbon source at a value of 3.

The reagents were premixed for 1 hour outside the reactor and prior to the addition of the iron additive. On the other hand, 13.52 g of iron (III) chloride powder or trichloride was premixed in 200 mL of deionized water slowly to ensure good dispersion of the iron, avoiding overheating of the sample. Once the two predispersions were obtained, both were added to the reactor by slowly introducing the iron predispersion over the urea and citrate predispersion under continuous stirring.

The reactions were carried out in a Parr Instruments stirred tank batch-type autoclave reactor. The reactions were carried out under agitation at 600 rpm with a turbine-type stirrer with two stirring levels with 4 blades.

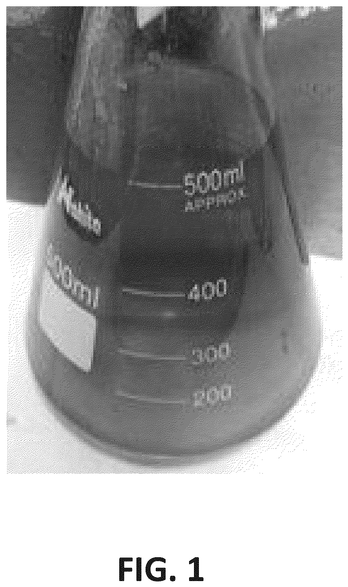

The temperature ramp was adjusted with a PID controller (Parr 4848 controller) to ensure a heating rate of 3°C/min until reaching a set temperature of 180°C after one hour of heating. The reaction was maintained for 16 hours at autogenous pressure (hermetic system) after which the reaction was rapidly cooled to room temperature. The system pressure at the set temperature was 40 bars. Once the product in suspension was recovered, it was filtered with a 0.22 µm syringe filter (with Nalgene PTFE material) and dried and stored for later use (Fig. 1).

*Biochar production stage from the pyrolysis process.*

Tree species *(Eucalyptus Globulus)* and forest waste from clearing the heather bush area *(Calluna Vulgaris)* were used as starting material.

The material was previously ground through a chipping equipment that allows obtaining pellets between 1 and 3 cm in size. This material was dried at room temperature with an overnight air flow until the humidity was reduced to below 10%. The dry material was then introduced into the pyrolysis reactor. The reactor used was a vertical fixed bed reactor (Hobersal). It is a reactor with a diameter of 7 cm and a length of 1500 mm with a homogeneous heating effect of 1000 mm and a lower and upper region of 250 mm. The material was supported on a porous ceramic disc covered with mineral fiber to filter the by-products resulting from liquefaction during pyrolysis.

A total of 500 g of dry and crushed material was fed into the reactor from the top, which was heated externally with an electric oven. Before feeding the raw material, a flow of nitrogen was introduced into the reactor with a gas flow rate always higher than 0.03 m/s.

Thermal treatment with slow temperature ramps of 5 K/min up to a set temperature of 400°C. The residence times used reached 30 minutes. The nitrogen atmosphere in the furnace was always pressurized below 5 bars, avoiding an increase in the residence time of nitrogen in the reactor. The vapors and gases obtained were evacuated during pyrolysis while the biochar was normally collected after cooling the system to room temperature. The process efficiency for the selected biomass material reaches up to 50% (for each batch about 250 g of biochar were obtained).

The collected material was then ground in a RETSCH ZM-300 ultracentrifugal mill that rotates at 20,000 rpm and passes the material through a 2 mm mesh sieve (maximum particle size obtained from grinding).

A surface activation treatment was then carried out to increase the specific surface area of the biochar obtained to 500 m²/g by means of mechanical exfoliation using ultrasound. For every 450 g of material obtained from the pyrolysis treatment, it was resuspended in 900 mL of deionized water and treated with an ultrasound probe for 30 min in an ice bath to prevent overheating. The ultrasound equipment was a BANDELIN SONOPULS HD3200 sonicator with a TT13 titanium tip operating at an amplitude of 20% and a frequency of 20 kHz. The pH of the biochar suspended in water was characterized as around 9.

### Impregnation stage

A total of 50 g of Fe-GQDs were resuspended in 100mL of distilled H₂O until the turbidity of the medium was eliminated. Always under agitation, the 450 g of biochar were slowly introduced until reaching a total volume of 1 L with a concentration of 10% wt of GQDs with respect to biochar. The suspension was used as a preconcentrate to formulate the final concentration of additive in the anaerobic fermentation reactor.

### Example 2 - Characterization of the Catalytic Material

The catalytic composition was thoroughly characterized to confirm the structural and electrochemical properties relevant to its performance in AD processes.

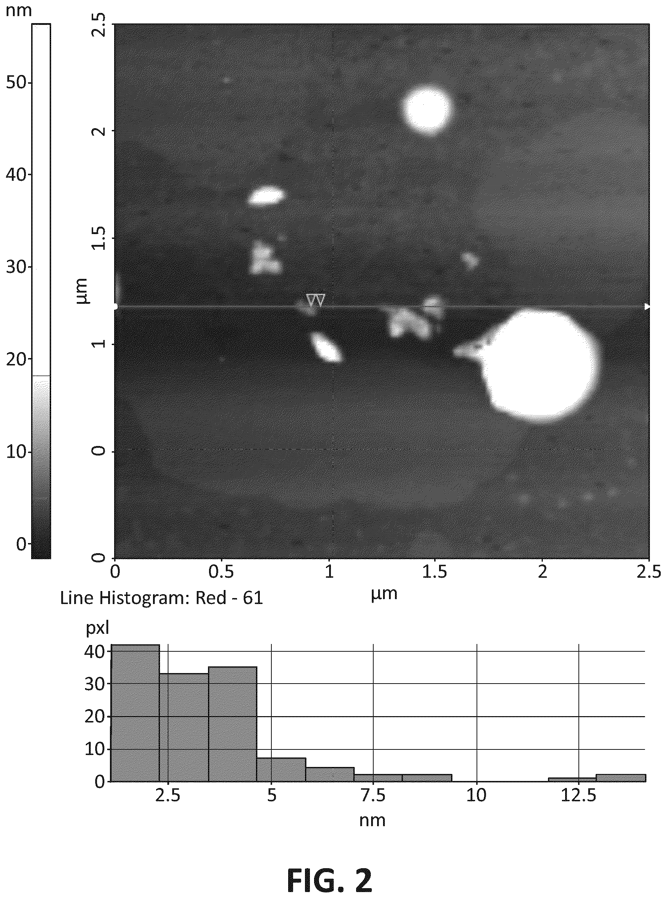

The Fe-GQDs nanostructures were characterized by atomic force microscopy or AFM (Fig. 2) to determine the size distribution. The results reports a well-defined size distribution below 5 nm, which favors their dispersion and enhances the efficiency of bacterial electrostimulation by increasing the active surface area available for redox interactions. This nanoscale size ensures high contact efficiency with microbial communities in the reactor, contributing to improved methanogenesis rates.

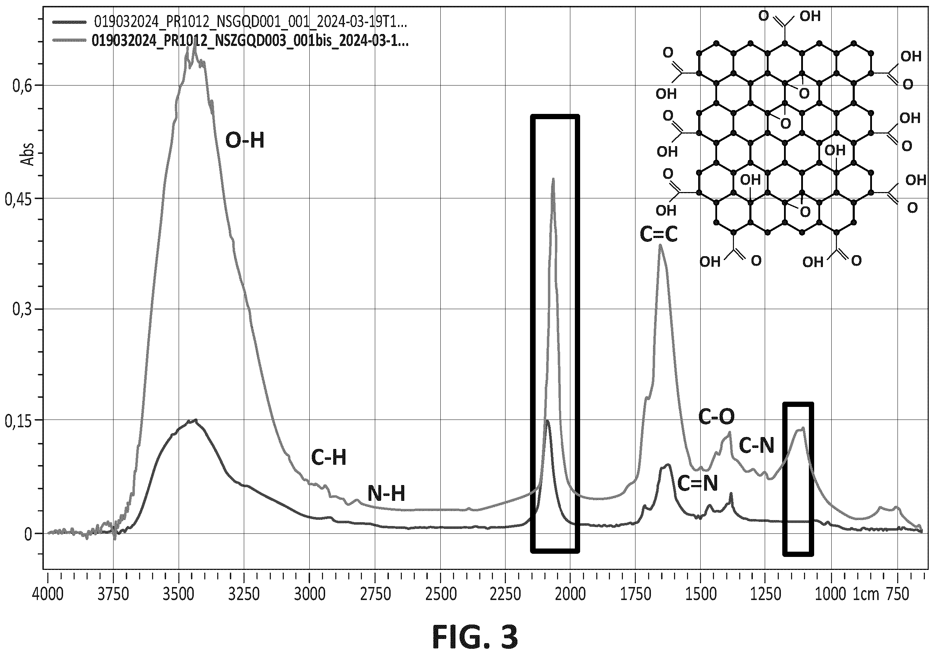

In addition, the study of chemical functional groups was carried out by means of infrared spectrum analysis or **FTIR.** Figure 3 allows the identification of structural modifications induced by iron doping, as evidenced by the shift and intensification of characteristic bands associated with oxygen-containing functional groups and metal-oxygen interactions. This functionalization is key to enhancing the redox reactivity of the Fe-GQDs, enabling them to serve as highly efficient electron mediators within the microbial consortia.

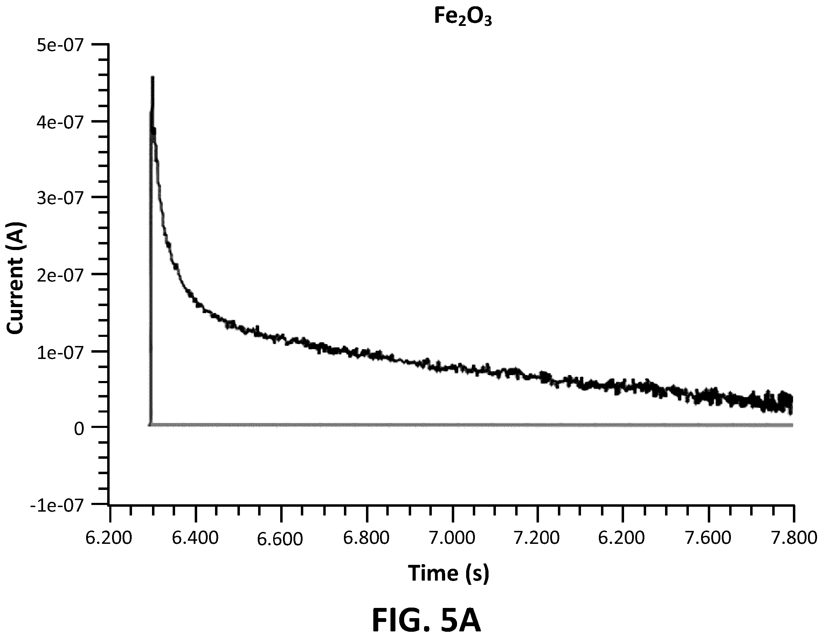

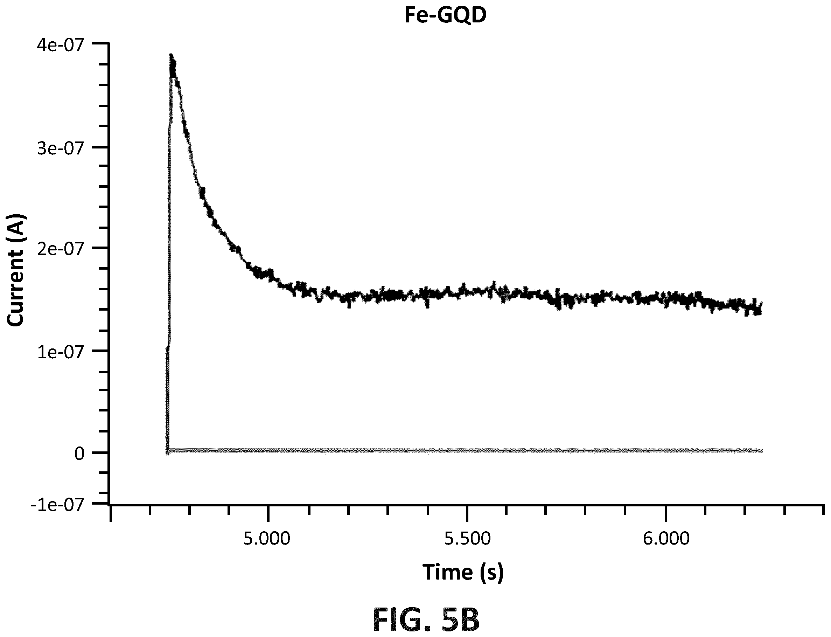

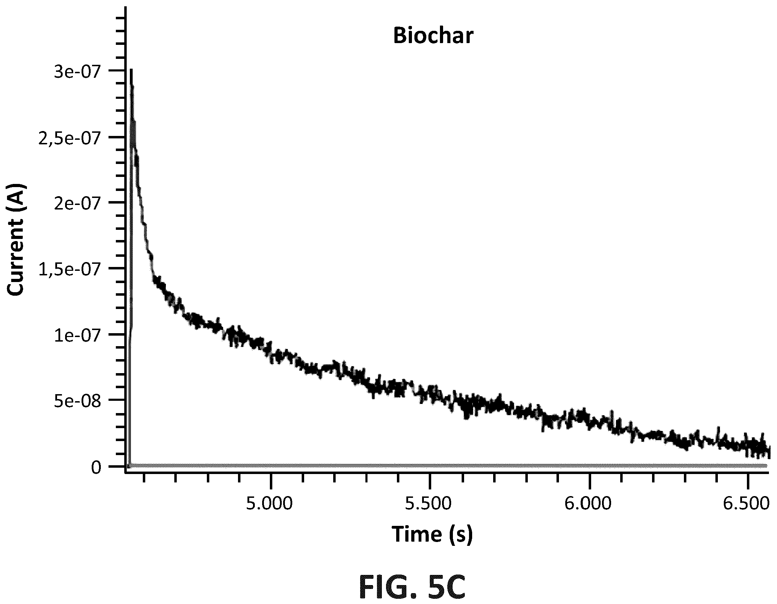

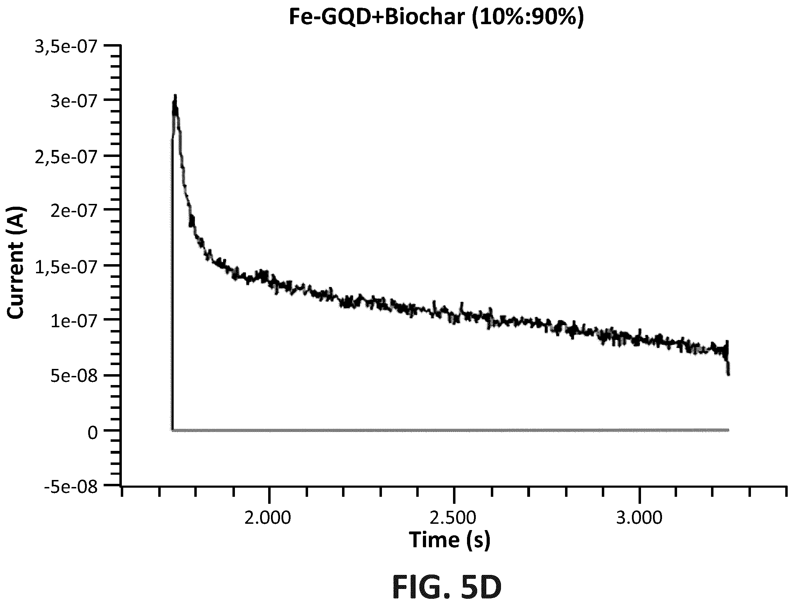

**Figure** 5 presents the chronoamperometric curves corresponding to different tested materials. The curve obtained for Fe-GQD/Biochar (10:90) shows a visibly larger area under the current-time curve compared to those of pristine biochar and Fe₂O₃, indicating a higher electron transfer activity. This visual trend suggests enhanced redox behavior, which is further evaluated and quantified in the catalytic performance tests described below in Example 3.

### Example 3 - Evaluation of Fe-GQD/Biochar Catalytic Activity

In order to assess the catalytic performance of the composition of the invention in the context of anaerobic fermentation, the EDC of the material of the invention was measured using a mediated electrochemical oxidation (MEO) technique. EDC measurement is widely recognized in the technical field as such electrochemical parameters predict the ability of a material to facilitate electron transfer processes, which are essential to promote microbial metabolism, particularly in systems where **DIET** is operative.

Numerous publications have reported a strong correlation between the electrochemical properties of carbonaceous or doped carbon-based materials (e.g. redox potential, conductivity, or Electron Donor/Acceptor Capacity (EDC/EAC values)) and their ability to catalyse the production of methane during AD processes *(*Z. Sun et al. The role of electrochemical properties of biochar to promote methane production in anaerobic digestion Journal of Cleaner Production, Volume 362,2022, 132296,ISSN 0959-6526*);* Y. Qin et al. Specific surface area and electron donating capacity determine biochar's role in methane production during anaerobic digestion. Bioresour Technol, 2020, 303:122919*].* The EDC, together with the EAC, constitutes the electron exchange capacity (EEC) of a material, and reflects its redox reactivity. In carbonaceous materials such as biochar or GQDs, these properties are linked to the presence of redox-active functional groups, such as oxygen-containing moieties or dopants (e.g. nitrogen, boron), that can undergo reversible oxidation or reduction *(*F. J. Chacón et al. Understanding, measuring and tuning the electrochemical properties of biochar for environmental applications. Rev. Environ. Sci. Biotechnol. 2017*).*

### Methods

MEO of Fe-GQDs/Biochar was carried out in a glassy carbon cylinder (volume: 9 mL; Sigradur G, HTW, Germany) serving as the working electrode (WE). The cylinder was filled with 6 mL of buffer solution (0.1 M phosphate and 0.1 M KCI, pH 7) and polarized to a potential of Eh = +0.61 V. A platinum wire was used as an auxiliary electrode, separated from the working electrode compartment by a porous glass capsule. An Ag/AgCl reference electrode (model) was used. All potentials are referenced to the standard hydrogen electrode (SHE).

Then, 130 µL of ABTS mediator solution (10 mM) was added to the cell, and current peaks were measured during continuous amperage measurements. After adding the mediator, the measured amperage was allowed to stabilize. Measurements were carried out with gentle magnetic stirring, and the current was allowed to stabilize at the working potential. The potentiostat was operated in chronoamperometry mode, measuring the electric current over time (25 minutes of measurement) at a constant voltage of 0.416 V (relative to the Ag/AgCl reference electrode) (Fig. 5).

The Fe-GQD/Biochar particles under study were resuspended in buffer solution (0.1 M phosphate and 0.1 M KCI, pH 7) at a concentration of 1 g/L using an ultrasonic probe and added in small volumes (60 µL per cell). The area under the curve of the current peaks versus time was then integrated and entered into the following equation: $EDC = \frac{\int \frac{{I}_{ox}}{F} d t}{{m}_{char}} ,$ wherein mchar is the mass of the added material (in grams), and F is Faraday's constant (96485 s·A/mol e⁻).

### Tested Materials and Results

The same test were carried out for the following materials:
- Iron oxide (Fe₂O₃) as a conventional reference catalyst,
- Fe-GQDs (Fe-doped graphene quantum dots),
- Pristine biochar, and
- the composition according to the invention: Fe-GQDs/Biochar (10:90 w/w).

The results are shown in the table below:

| **Sample** | **Mass added (µg)** | **Integrated area (A·s)** | **EDC (µmol e⁻/g)** |
|---|---|---|---|
| Fe₂O₃ (reference) | 60 | 0.000127953 | 22.10 |
| Fe-GQDs | 60 | 0.000256664 | 44.33 |
| Biochar | 60 | 0.000102633 | 17.72 |
| Fe-GQDs/Biochar (10:90%) | 60 | 0.000166928 | 28.83 |

These results confirm that the catalytic composition of the invention, Fe-GQD/Biochar (10:90), exhibits a substantially higher EDC compared to pristine biochar (17.72 µmol e⁻/g) and Fe₂O₃ (22.10 µmol e⁻/g) alone.

This demonstrates a clear synergistic effect between the Fe-GQDs and the porous carbon support, validating the proposed formulation as an effective and improved catalytic additive for enhancing methanogenesis in AD.

Although the EDC of pure Fe-GQDs particles is higher (44.33 µmol e⁻/g), the composition of the invention achieves an excellent balance between performance and iron content, as it provides enhanced redox activity while using a significantly lower amount of iron. Therefore, the enhanced EDC is translated into an improved capacity to catalyze the anaerobic fermentation reaction towards the generation of methane with a lower consumption of iron and a reduction in the potential toxicity of the additive on bacterial colonies.

The results validate the formulation as a highly efficient, stable, and sustainable additive for AD. Importantly, the reduced iron content mitigates toxicity concerns while maintaining catalytic effectiveness, making the material especially suitable for large-scale applications in biogas production systems.

These findings demonstrate that the Fe-GQDs/Biochar composite not only exhibits favorable nanoscale and redox characteristics but also translates these features into superior catalytic performance under conditions relevant to AD. Its improved EDC, combined with a reduced iron content, offers a more sustainable and efficient alternative to conventional catalysts such as Fe₂O₃ or NZVI, particularly in systems relying on DIET.

## Claims

1. A catalytic composition with electrostimulation capacity of methanogenic bacteria comprising:
a. iron-doped graphene quantum dots (Fe-GQDs) nanostructures, and
b. a carbon porous structure,
wherein the Fe-GQDs nanostructures are impregnated inside the porous internal surface and the external surface of the carbon porous structure, and wherein the carbon porous structure has a high surface area ranging from 200 m²/g to 1000 m²/g.

2. The catalytic composition according to claim 1 wherein the Fe-GQDs nanostructures have a contain less than 10 wt% of Fe, relative to the total weight of the catalytic composition.

3. The catalytic composition according to claim 1 wherein the Fe-GQDs nanostructures have a particle size of less than 10 nm.

4. The catalytic composition according to claim 1 wherein Fe-GQDs are present in an amount ranging from 10 wt% to 20 wt%, relative to the weight of the carbon porous structure.

5. The catalytic composition according to any one of claims 1 to 4 wherein the carbon porous structure is biochar or charcoal.

6. Use of the catalytic composition according to any of claims 1 to 5 as an additive in Anaerobic Digestion (AD) processes for methane production.

7. Method of obtaining the catalytic composition of any of claims 1 to 5 comprising the steps of:
i. Hydrothermal synthesis of Fe-GQDs nanostructures,
ii. Generation of carbon porous structures by pyrolysis, and
iii. Impregnation of the Fe-GQDs inside the internal and external surface of the carbon porous structures.

8. Method according to claim 7 wherein in step iii) the Fe-GQDs nanostructures are incorporated into the carbon porous structure in a proportion ranging from 10 wt% to 20 wt%, relative to the weight of the carbon porous structure.

9. Method to promote methane production during AD of biomass comprising: a) obtaining biomass as starting material of the AD; b) adding the catalytic composition of any of claims 1 to 5 to a biomass AD reactor; and c) collecting the produced methane.

10. Method according to claim 9 wherein, in step b), the catalytic composition is added to the biomass AD reactor in a concentration comprised between 1 and 5 g/L.

11. Method according to claim 9 wherein the AD process is implemented in batch or semicontinuous mode.

12. Method according to claim 11, wherein the AD process is implemented in batch mode and carried out under reactor conditions comprising a temperature between 35 and 40 °C and a pH between 7 and 7.5, for a period of at least 4 days.

13. The method according to claim 9 wherein at least one from the carbon porous structure and the Fe-GQDs nanostructures of the catalytic composition is obtained from the feedstock biomass used as starting material in the AD process.
